# FASCICULE DE BREVET EUROPEEN

(11) **EP 0 624 382 B1**
(45) Date de publication et mention de la délivrance du brevet: **02.09.1998**
(21) Numéro de dépôt: 94401017.2
(22) Date de dépôt: 09.05.1994
(51) Int. Cl.: A61M 39/00

(54) **Pince péristaltique de sécurité**
Peristaltische Sicherheitsklemme
Peristaltic security clamp

(30) Priorité: 13.05.1993 FR 9305762
(43) Date de publication de la demande: 17.11.1994
(73) Titulaire: ASSOCIATION POUR L'ESSOR DE LA TRANSFUSION SANGUINE DANS LA REGION DU NORD, 59000 Lille (FR)
(72) Inventeur: de Baudus, Jacques, F-59800 Lille (FR); Sanchez, Enriqué, F-62220 Carvin (FR); Depoortere, Jacques, F-59493 Villeneuve d'Ascq (FR); Bouzin, Georges, F-59491 Villeneuve d'Ascq (FR)
(74) Mandataire: Lhuillier, René

(56) Documents cités:
- EP-A- 0 115 276
- WO-A-89/10147
- FR-A- 2 379 290
- US-A- 4 758 228
- US-A- 4 976 687

## Description

L'invention concerne une pince péristaltique de sécurité destinée à équiper des appareils portables fonctionnant sur batterie, afin de commander l'écoulement d'un liquide devant rester stérile, par exemple du sang d'un appareil de prélèvement automatique.

Un appareil de ce genre doit impérativement être pourvu d'un mécanisme pouvant interrompre l'admission du sang prélevé quand le volume recueilli est atteint. Dans certaines installations connues, cette interruption s'effectue en amont de la poche de sang, sur le tube souple de prélèvement, grâce à une vanne qui ferme ledit conduit et qui est commandée par le gonflement de la poche de sang dans sa dernière phase de remplissage. Pour cela on utilise une tige réagissant à la déformation de la poche, qui pince le conduit souple et interrompt le débit. Ce système présente l'inconvénient d'abîmer rapidement le tube du fait que la force de pincement est souvent trop importante, au risque de compromettre la stérilité du produit transporté. Pour éviter cela on pourrait mettre en oeuvre un dispositif de pincement commandé électriquement, du genre d'une électrovanne, mais sa consommation d'énergie rendrait son emploi difficilement acceptable dans des appareils portables devant fonctionner sur batterie et consommer le moins d'énergie possible.

On connait par le FR-A-2 379 290 une pince péristaltique de sécurité comportant une came, entraînée en rotation par un moteur et un réducteur qui agit sur un tube souple par l'intermédiaire d'un embout. Mais le mécanisme à came est extérieur au corps principal du dispositif, il est complexe et se prête mal aux appareils portables.

Afin de remédier à ces inconvénients, la Demanderesse a été conduite à mettre au point une nouvelle pince de sécurité destinée notamment aux appareils pince de sécurité destinée notamment aux appareils portables de prélèvement de sang, mais aussi à tous appareils autonomes disposant de tubes souples de transfert de liquide, pince qui consomme très peu d'énergie et qui appuie sur le tube de façon péristaltique, évitant ainsi tout risque de détérioration.

L'invention a donc pour objet une pince péristaltique de sécurité notamment pour appareil portable de prélèvement automatique de sang comportant une came entrainée en rotation par un moteur et un réducteur, dans laquelle la came tourne à l'intérieur d'un bâti en forme de U et pince directement un tube souple contre une bordure du bâti, un jeu de capteurs à effet Hall détectant la position de la came et commandant le fonctionnement du moteur.

Selon une caractéristique particulière de l'invention deux capteurs à effet Hall sont montés de façon diamétralement opposés, de part et d'autre de l'axe, sur le corps du réducteur. En outre, un petit aimant est monté sur une molette à l'extrémité inférieure de l'axe de la came.

D'autres caractéristiques et avantages de l'invention apparaitront à la lecture de la description qui va suivre d'un exemple non limitatif de réalisation dans lequel il est fait référence au dessin annexé qui représente :
figure 1 une vue schématique en élévation de la pince péristaltique ;
figure 2 une vue selon II-II de la figure 1 ;

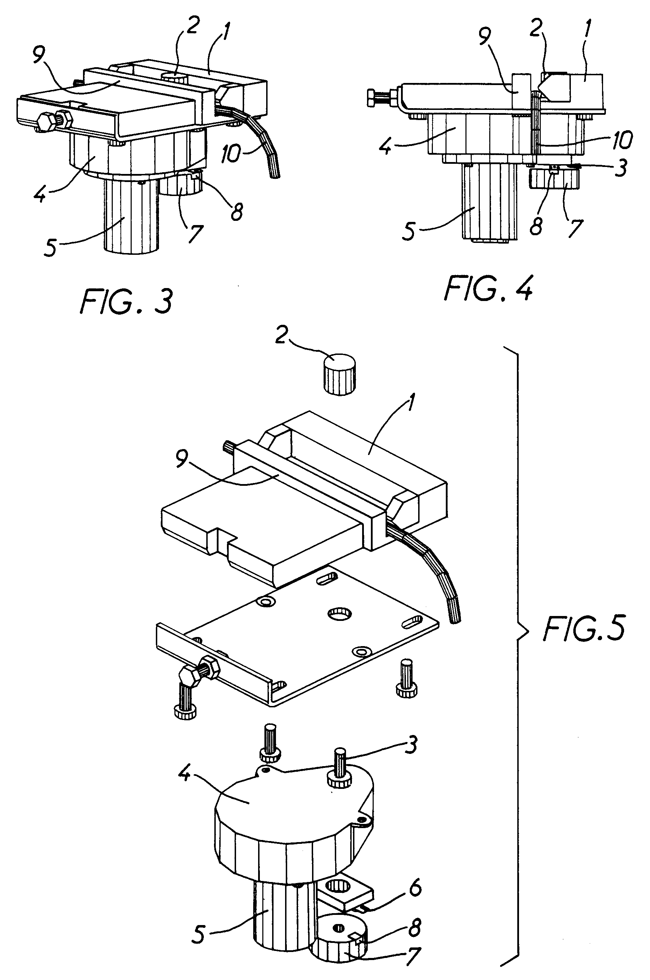

figures 3 et 4, des vues générales, respectivement en perspective et en élévation de la pince ;
figure 5, une vue éclatée en perspective de la pince.

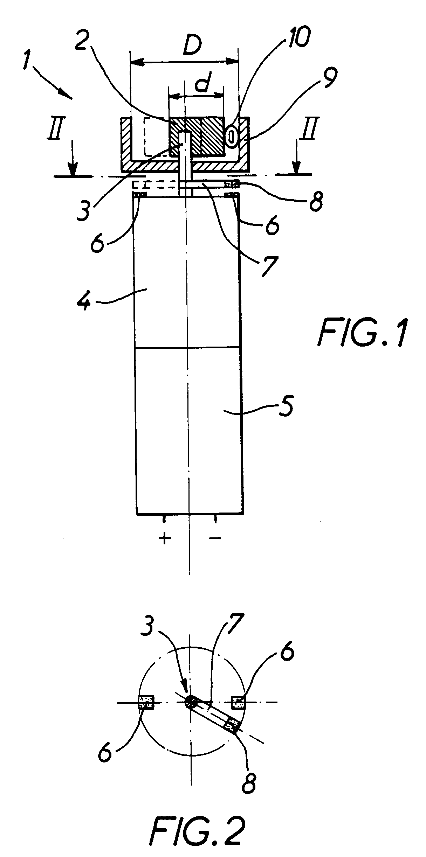

La pince péristaltique représentée sur les figures est essentiellement constituée d'un bâti 1 ou d'une coupelle ayant une section en forme de U avec des rebords 9, et d'une came 2 ayant la forme d'un petit cylindre, logée à l'intérieur dudit bâti. Le diamètre d de la came représente sensiblement la moitié de la largeur du bâti ou du diamètre D de la coupelle qui constitue le bâti. La came 2 est fixée de façon excentrique sur l'extrémité supérieure de l'axe vertical 3 traversant le fond du bâti. L'axe 3 est en sortie d'un réducteur 4 à moment d'inertie élevé dont le train d'engrenage est entraîné par un moteur 5 du genre d'un micromoteur 12 volts à aimant permanent tournant à environ 20000 tours/minutes. Sur la face inférieure du réducteur 4 sont fixés deux capteurs 6 à effet Hall, diamétralement opposés. Sur l'extrémité inférieure de l'axe 3, est montée une molette 7 sur laquelle a été collé un petit aimant 8. On voit à la figure 2 que cet aimant, entrainé en rotation avec l'axe 3, suit une trajectoire circulaire, qui peut l'amener en surplomb des capteurs 6.

A l'intérieur du bâti 1 est placé le tube souple 10 acheminant le sang vers la poche de recueil. La figure 1 montre ledit tube souple pincé par la came contre le rebord 9 du bâti. La came dessinée en traits pleins correspond à la position "fermée" de la pince. On a représenté en pointillés la position "ouverte" de la came, à 180° de la précédente. L'aimant 8 occupe alors la position inverse également visible en pointillés.

Dès que le logiciel commande le moteur 5, la came est entrainée rapidement de la position "ouverte" à la position "fermée" par rotation de 180° de l'axe 3. La simultanéité de déplacement de la molette 7 portant l'aimant 8 permet la lecture de la position de la came et l'arrêt du moteur dès qu'un signal apparaît sur le capteur à effet Hall 6, placé du côté position "fermée". En fait, pour éviter que la came ne dépasse cette position "fermée" à cause de l'inertie du train d'engrenages du réducteur, le logiciel à commande rapide interrompt l'alimentation du moteur 5 et inverse aussi pendant un temps très court (d'environ 100 m S) la polarité de l'alimentation, dès que l'aimant 8 est décelé par le capteur à effet Hall. Cette inversion de sens freine la came et assure son immobilisation en position "fermée". On a ainsi un pincement progressif du tube qui évite sa détérioration. Le moteur reste hors tension quand la pince est en position fermée ; la came 2 en effet ne peut tourner et quitter cette position du fait du moment d'inertie du réducteur qui lui oppose une force suffisante. Il n'y a donc aucune consommation d'énergie quand la pince est en position "fermée" et l'on comprend tout l'intérêt d'une telle disposition dans un appareil portable autonome.

Après le prélèvement et le débranchement du tube, le mouvement de la came est commandé en sens inverse et celle-ci revient dans la position initiale pince "ouverte" décelée par le capteur 6 correspondant.

## Revendications

1. Pince péristaltique de sécurité notamment pour appareil portable de prélèvement automatique de sang comportant une came entraînée en rotation par un moteur (5) et un réducteur (4), caractérisée en ce que la came (2) est agencée pour tourner à l'intérieur d'un bâti (1) en forme de U et pincer directement un tube souple (10) contre une bordure (9) du bâti, et en ce qu'un jeu de capteurs (6) à effet Hall détecte la position de la came et commande le fonctionnement du moteur.

2. Pince péristaltique selon la revendication 1 caractérisée en ce que deux capteurs (6) à effet Hall sont montés de façon diamétralement opposée sur la face inférieure du réducteur (4).

3. Pince péristaltique selon la revendication 1 caractérisée en ce qu'un petit aimant (8) se trouve sur une molette (7) à l'extrémité inférieure de l'axe (3).

## Claims

1. Peristaltic security clamp especially for a portable automatic blood-taking apparatus, comprising a cam that is entrained in rotation by a motor (5) and a reducer (4), characterised in that the cam (2) is arranged to rotate inside a U-shaped structure (1) and to pinch directly a flexible tube (10) against a rim (9) of the structure, and in that a set of Hall effect sensors (6) detects the position of the cam and controls the operation of the motor.

2. Peristaltic clamp according to claim 1, characterised in that two Hall effect sensors (6) are mounted diametrically opposite to one another on the lower face of the reducer (4).

3. Peristaltic clamp according to claim 1, characterised in that a small magnet (8) is located on a wheel (7) at the lower end of the axle (3).

## Patentansprüche

1. Peristaltische Sicherheitsklemme, insbesondere für ein tragbares Gerät zur automatischen Blutentnahme, eine durch einen Motor (5) und ein Untersetzungsgetriebe (4) in Drehung versetzte Kurvenscheibe umfassend,
**dadurch gekennzeichnet,**
daß die Kurvenscheibe (2) gestaltet ist, um sich in einem U-förmigen Gehäuse (1) zu drehen und einen Schlauch (10) direkt gegen einen Rand (9) des Gehäuses zu quetschen, und dadurch, daß ein Satz Halleffekt-Sensoren die Stellung der Kurvenscheibe detektiert und den Motorbetrieb steuert.

2. Peristaltische Klemme nach Anspruch 1, dadurch gekennzeichnet, daß zwei Halleffekt-Sensoren (6) diametral entgegengesetzt auf der Unterseite des Untersetzungsgetriebes (4) angebracht sind.

3. Peristaltische Klemme nach Anspruch 1, dadurch gekennzeichnet, daß ein kleiner Magnet (8) sich an einem Rädchen (7) am unteren Ende der Achse (3) befindet.
